# FASCICULE DE BREVET EUROPEEN

(11) **EP 3 830 073 B1**
(45) Date de publication et mention de la délivrance du brevet: **01.05.2024**
(21) Numéro de dépôt: 19745635.3
(22) Date de dépôt: 02.08.2019
(51) Int. Cl.: C07C 231/02, C07C 233/69

(54) **PROCÉDÉ DE PRÉPARATION MONOTOPE DE COMPOSÉS ORGANO-IODÉS INTERMÉDIAIRES À LA SYNTHÈSE DU IOVERSOL**
VERFAHREN ZUR MONOTOPISCHEN HERSTELLUNG VON IODORGANISCHEN ZWISCHENVERBINDUNGEN ZUR HERSTELLUNG VON IOVERSOL
PROCESS FOR THE MONOTOPIC PREPARATION OF INTERMEDIATE ORGANO-IODINATED COMPOUNDS FOR THE SYNTHESIS OF IOVERSOL

(30) Priorité: 02.08.2018 FR 1857242
(43) Date de publication de la demande: 09.06.2021
(73) Titulaire: Guerbet, 93420 Villepinte (FR)
(72) Inventeur: PELLINGHELLI, Stéphan, 95820 BRUYERES SUR OISE (FR); PETTA, Myriam, 95160 MONTMORENCY (FR)
(74) Mandataire: Lavoix
(86) Numéro de dépôt international: PCT/EP2019/070869
(87) Numéro de publication internationale: WO 2020/025787

(56) Documents cités:
- WO-A1-2012/175903
- GB-A- 2 496 971

## Description

La présente invention concerne un procédé de préparation de composés organo-iodés, ainsi que ses intermédiaires de préparation. Plus particulièrement, la présente invention concerne un procédé de préparation de composés organo-iodés utiles en tant qu'intermédiaires de préparation dans la synthèse du produit de contraste iodé ioversol (Optiray^{®}).

loversol est décrit comme un produit de contraste non-ionique dans le brevet US 4,396,598.

Actuellement, la majorité des procédés de synthèse des produits de contraste iodés et notamment celui concernant le ioversol, utilisent comme produit intermédiaire le dichlorure d'acide 5-amino-2,4,6-triiodoisophtalique (aussi appelé DiCOCI), de formule suivante :

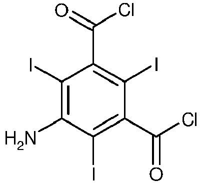

Lors de la synthèse de produits de contraste iodés, il est nécessaire de réaliser de longues étapes de séparation et de purification pour aboutir à des intermédiaires de synthèse avec un bon degré de pureté. Ces étapes augmentent considérablement le temps de réalisation de la synthèse et augmentent donc ainsi le coût de mise en oeuvre des procédés de préparation des produits de contraste.

L'une des étapes de fabrication du DiCOCI est une étape de chloruration (aussi appelée chloration) de l'acide 5-amino-2,4,6-triiodoisophtalique (AATI) avec un agent chlorurant tel que le chlorure de thionyle, appelé aussi dichlorure de thionyle (SOCl₂). Cette chloruration est une étape lente (plus de 7 heures 30 de temps de réaction) et consommatrice en énergie, puisqu'elle est réalisée à une température élevée de plus de 48°C. Un excès important de l'agent chlorurant par rapport à l'AATI permet d'obtenir une cinétique plus élevée mais l'utilisation de cet excès n'est pas acceptable d'un point de vue industriel et environnemental. En effet, le chlorure de thionyle, au contact de l'eau, libère du chlorure d'hydrogène (HCl) et du dioxyde de soufre (SO₂), qui sont des gaz corrosifs et irritants. L'utilisation de catalyseurs de cette réaction de chloruration a été préconisée de façon à pouvoir réduire la quantité de SOCl₂ utilisée tout en obtenant un bon rendement industriel.

Les procédés de préparation de DiCOCI utilisés ont également comme inconvénient de générer une quantité importante d'effluents, du fait de l'utilisation de grandes quantités d'eau au moment de l'hydrolyse « précipitante » du DiCOCI obtenu par l'étape de chloruration. Par exemple, dans la demande EP 0 794 937 est décrit l'ajout de 22,2 à 33 équivalents d'eau par équivalent d'AATI (Exemples 1 à 3) ou dans la demande EP 0 773 925 est décrit l'ajout de 120 équivalents d'eau (voir exemple 1-E) pour hydrolyser le chlorure de thionyle. Enfin, ces procédés nécessitent la réalisation d'étapes de purification par précipitation, filtration et séchage, afin d'assurer une réactivité optimale et un rendement compétitif dans les étapes suivantes. Il faut aussi noter que l'étape de séchage peut s'avérer dangereuse puisqu'elle présente un risque industriel de dégradation exothermique et doit donc être réalisée dans des conditions très maitrisées et contraignantes.

Suite à cette étape de chloruration, le DiCOCI est acylé. Cette étape est très longue puisqu'elle peut durer plusieurs dizaines d'heures (jusqu'à 70h parfois). Elle implique également des étapes de purification et d'isolement par essorage et séchage pour obtenir un intermédiaire de synthèse de produits de contraste iodés. L'étape de séchage présente le risque souligné ci-dessus. De plus, cette étape implique l'utilisation d'excès importants de certains réactifs qui, peuvent pour certains, s'avérer couteux que ce soit à leur achat ou même à leur synthèse. La manipulation de certains de ces réactifs (par exemple le DiCOCI) peut aussi poser des problèmes du fait de leur granulométrie. Pour cette seule étape d'acylation, on obtient des rendements limités à environ 87,5%. Le rendement de l'étape de chloruration est d'environ 90,5%. Le rendement de la combinaison des étapes de chloruration et d'acylation est alors d'environ 79,2%.

Il est également connu des procédés de préparation impliquant une acylation suivie d'une chloruration qui présentent les mêmes inconvénients que cités précédemment.

En particulier, la demande WO 2012/175903 décrit l'obtention d'un AATI acylé, obtenu à partir d'un large excès d'agent acylant. Ce composé intermédiaire acylé est ensuite isolé, filtré et séché avant d'être chloruré.

Par conséquent, il existe un besoin pour un procédé de préparation de produits de contraste iodés amélioré. Plus particulièrement, il existe un besoin pour un procédé de préparation de produits de contraste iodés applicable à l'échelle industrielle, plus économique, rapide et sûr.

Plusieurs brevets décrivent des procédés de préparation du ioversol ou de certains de ses intermédiaires de synthèse : US 4,997,983, EP 0 484 328 (procédé de préparation de l'AATI), EP 0 598 751 (procédé de préparation d'un intermédiaire de synthèse du ioversol à partir d'un autre composé que l'AATI, sans utilisation de chlorure d'acétoxyacétyle (aussi appelé 2-chloro-2-oxoéthyl acétate ou AAC) et en utilisant du DMAC et du chlorure de chloroacétyle (aussi appelé CAC), EP 0 640 067 (procédé de préparation du ioversol à partir d'un autre composé que l'AATI). D'autres décrivent des procédés de purification de ces composés : EP 0 618 836, EP 0 646 021, EP 0 863 782, EP 1 551 522, EP 0 700 377, EP 0 470 247, EP 0 907 395, EP 0 515 480.

D'autres brevets décrivent certaines parties de cette synthèse : EP 2 281 807 et EP 2 277 846.

La présente invention a pour but de fournir un procédé de préparation de produits organo-iodés, et plus particulièrement d'intermédiaires de synthèse du produit de contraste iodé ioversol, permettant de pallier aux inconvénients mentionnés ci-dessus.

La présente invention a également pour but de fournir un procédé de préparation de composés organo-iodés applicable à l'échelle industrielle, en particulier un procédé sûr, rapide, économique et acceptable d'un point de vue environnemental.

La présente invention a pour but de fournir un procédé de préparation de composés organo-iodés intermédiaires de synthèse du produit de contraste iodé ioversol présentant un bon rendement, et notamment un meilleur rendement par rapport aux procédés connus.

La présente invention concerne donc un procédé de préparation d'un composé organo-iodé comprenant les étapes suivantes :
a) acylation de l'acide 2,4,6-triiodo-5-aminoisophtalique de formule (A) suivante : 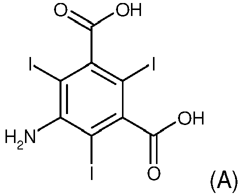
   pour obtenir un composé intermédiaire Ya ;
   puis
b) chloruration du composé intermédiaire Ya pour obtenir un composé intermédiaire organo-iodé Yb ;
   puis
c) amidification du composé intermédiaire organo-iodé Yb, en présence d'une base inorganique, pour obtenir un composé intermédiaire Yc ;
   puis
d) déprotection du composé intermédiaire Yc,
   les étapes a), b), c) et d) étant effectuées sans isolement des composés Ya et Yc.

De façon surprenante, les inventeurs ont mis en oeuvre un procédé monotope permettant de réaliser les étapes a) d'acylation, b) de chloruration, c) d'amidification et d) de déprotection sans nécessité d'isolement des composés intermédiaires Ya et Yc. L'acylation de l'AATI est alors réalisée puis est directement suivie par la chloruration dudit AATI acylé sans isolement du composé intermédiaire Ya, elle-même suivie d'une amidification du composé Yb, de préférence sans purification du composé intermédiaire Yb et de la déprotection du composé Yc sans isolement du composé intermédiaire Yc.

La succession des étapes a) d'acylation, b) de chloruration, c) d'amidification et d) de déprotection telle que selon l'invention est de préférence réalisée dans un même milieu réactionnel, et correspond donc à un enchainement monotope (également appelé « one pot » en anglais).

Le procédé de préparation selon l'invention permet avantageusement d'éviter les étapes de séparation et de purification des composés intermédiaires comme les composés intermédiaires de synthèse Ya et Yc : l'utilisation des solvants de précipitation ou de lavage est réduite ou évitée, ainsi que le traitement des eaux mères correspondantes. Le procédé de préparation selon l'invention est donc plus économique, plus rapide et plus respectueux de l'environnement.

Généralement, le procédé est exempt d'ajout d'un non-solvant du composé intermédiaire Ya pendant l'étape a) et entre les étapes a) et b). Par « non-solvant du composé intermédiaire », on entend un solvant dans lequel le composé intermédiaire n'est pas soluble (solubilité inférieure à 0,1 g/L, voire 0,01 g/L, à 25°C) et qui est susceptible d'induire la précipitation du composé intermédiaire lorsque le non-solvant est introduit dans le milieu réactionnel. Une solution aqueuse, comme de l'eau, est un exemple de non-solvant du composé intermédiaire Ya.

Le solvant de l'étape a) comprend du diméthylacétamide, le procédé peut comprendre, entre les étapes a) et b), l'ajout d'un solvant du composé intermédiaire Ya au milieu réactionnel obtenu à la fin de l'étape a), afin de le fluidifier. En effet, l'étape b) suivante, qui est réalisée en présence d'agent chlorurant, conduit souvent à la formation de complexes de Vilsmeier entre l'agent chlorurant et le diméthylacétamide, qui peuvent épaissir le milieu réactionnel en cristallisant. Ce solvant du composé intermédiaire Yb est typiquement un des solvants décrits ci-dessous (de préférence ceux exempts de fonction amide) pour les étapes a), b), c) et/ou d), et est de préférence du carbonate de propylène.

Le procédé peut comprendre, à la fin de l'étape b) :
- l'ajout d'un non-solvant du composé intermédiaire Yb dans le milieu réactionnel obtenu à la fin de l'étape b), ce par quoi le composé intermédiaire Yb précipite dans le milieu réactionnel et un milieu réactionnel comprenant une phase liquide et un précipité est obtenu,
- la séparation du précipité et de la phase liquide, par exemple par une filtration, une décantation ou une centrifugation, puis
- la dissolution du précipité dans un solvant du composé intermédiaire Yb pour obtenir une solution qui est celle utilisée pour mettre en oeuvre l'étape c).
Le non-solvant du composé intermédiaire Yb est généralement une solution aqueuse, de préférence une solution aqueuse d'acétate d'ion métallique alcalin, tel que l'acétate de sodium. Le solvant du composé intermédiaire Yb est typiquement un des solvants décrits ci-dessous pour les étapes a), b), c) et/ou d).

Le procédé est de préférence exempt de purification du composé intermédiaire Yb. Typiquement, lorsque les trois étapes d'ajout d'un non-solvant/de séparation du précipité/de dissolution du précipité sont mises en oeuvre, le précipité ne subit aucune purification entre la séparation du précipité et de la phase liquide et sa dissolution dans un solvant du composé intermédiaire Yb, et en particulier pas de recristallisation. Le procédé selon l'invention est applicable à l'échelle industrielle et permet notamment d'obtenir des rendements cumulés d'au moins 80% pour l'enchainement monotope des étapes a), b), c) et d).

Le procédé de préparation tel que selon l'invention a également comme avantage de générer des intermédiaires solubles (par « solubles », on entend que le procédé ne génère pas de cristaux).

### Définitions

Le procédé décrit dans la présente demande comprend un enchainement, de préférence monotope :
- des étapes a) et b) dans lequel le composé intermédiaire Ya issu de l'acylation (étape a)) n'est pas isolé avant de procéder à la chloruration (étape b)), et/ou
- des étapes b) et c) dans lequel le composé intermédiaire Yb issu de la chloruration (étape b)) n'est pas purifié avant de procéder à l'amidification (étape c)), et/ou
- des étapes c) et d) dans lequel le composé intermédiaire Yc issu de l'amidification (étape c)) n'est pas isolé avant de procéder à la déprotection (étape d)).

Dans un mode de réalisation, dans le procédé,
- le composé intermédiaire Ya issu de l'acylation (étape a)) n'est pas isolé avant de procéder à la chloruration (étape b)),
- le composé intermédiaire Yb issu de la chloruration (étape b)) n'est pas purifié avant de procéder à l'amidification (étape c)), et
- le composé intermédiaire Yc issu de l'amidification (étape c)) n'est pas isolé avant de procéder à la déprotection (étape d).

Dans un mode de réalisation, dans le procédé :
- le composé intermédiaire Ya issu de l'acylation (étape a)) n'est pas séparé du milieu réactionnel obtenu à la fin de l'étape a) avant de procéder à la chloruration (étape b)),
- le composé intermédiaire Yb n'est pas séparé du composé intermédiaire Ya éventuellement présent dans le milieu réactionnel à la fin de l'étape b) avant de procéder à l'étape d'amidification (étape c)), et
- le composé intermédiaire Yc issu de l'amidification (étape c)) n'est pas séparé du milieu réactionnel obtenu à la fin de l'étape c) avant de procéder à la déprotection (étape d)).

Un procédé de préparation ou un enchainement de réactions monotope est un procédé/enchainement dans lequel un intermédiaire de synthèse, par exemple l'AATI, subit plusieurs réactions successives et/ou simultanées dans son milieu réactionnel, en évitant les étapes de séparation et de purification des composés intermédiaires (procédé exempt de séparation et purification des composés intermédiaires Ya et Yc, et de préférence exempt de purification du composé intermédiaire Yb).

Par composé « organo-iodé », on entend un composé organique comprenant au moins un atome de carbone et au moins un atome d'iode, par exemple 1, 2, 3, 4 ou 5 atomes d'iode, de préférence 3. Ledit composé organique comprend éventuellement un ou plusieurs atome(s) d'hydrogène, d'oxygène, d'azote, de soufre, de phosphore, d'halogène ou une combinaison de ces atomes. De préférence, le composé organo-iodé comprend un ou plusieurs atomes d'hydrogène (composé hydrocarboné), d'oxygène, d'azote et éventuellement de chlore.

Par « acylation », on entend une réaction chimique au cours de laquelle un groupement acyle est ajouté à un composé organique tel que l'AATI, par l'action d'un agent acylant.

Par « chloruration », également appelée chloration, on entend la substitution d'un atome et/ou d'un groupe d'atomes d'un composé organique par un atome de chlore, de préférence la substitution d'un groupement hydroxyle (-OH) par un atome de chlore (-Cl), par l'action d'un agent chlorurant ou chlorant, plus préférentiellement la double substitution des groupements hydroxyles (-OH) présents sur deux fonctions acides carboxyliques par un atome de chlore (-Cl).

Par « amidification », également appelée « amidation » en anglais, on entend la combinaison d'une amine à un groupement chimique d'un composé.

Par « déprotection », on entend l'élimination, typiquement par un agent déprotecteur, d'un groupement fonctionnel introduit dans la molécule à partir d'une fonction chimique pour masquer tout ou partie de sa réactivité.

Selon la présente invention, l'expression « milieu réactionnel » désigne le milieu dans lequel ont lieu les étapes a) d'acylation, b) de chloruration, c) d'amidification et d) de déprotection. Selon un mode de réalisation, ledit milieu réactionnel comprend au moins un solvant et au moins un réactif tel que l'AATI et/ou un agent acylant et/ou un agent chlorurant et/ou une amine et/ou un agent déprotecteur.

Par « isolement », on désigne :
- la séparation d'un composé intermédiaire Ya, Yb et/ou Yc de(s) l'autre(s) composé(s) organo-iodé(s) éventuellement présent(s) dans le milieu réactionnel,
- voire la séparation d'un composé intermédiaire Ya, Yb et/ou Yc du milieu réactionnel,
éventuellement suivie de sa(leurs) purification(s). Les méthodes de séparation et/ou de purification d'un composé organo-iodé sont connues de l'homme du métier. Peuvent par exemple être citées la filtration, la chromatographie (par exemple sur silice greffée ou non), la centrifugation, l'extraction par solvant, la cristallisation, l'adsorption (par exemple sur charbon) et la distillation. Le procédé selon l'invention est exempt d'une étape de séparation du composé intermédiaire Ya du milieu réactionnel obtenu à la fin de l'étape a) et d'une étape de séparation du composé intermédiaire Yc du milieu réactionnel obtenu à la fin de l'étape c). Le procédé est généralement exempt de filtration du composé Ya entre les étapes a) et b) (pas de séparation du composé Ya) et/ou il est généralement exempt de filtration du composé Yc entre les étapes c) et d) (pas de séparation du composé Yc). Ci-après sont donnés des exemples de non séparation d'un composé intermédiaire Ya, Yb et/ou Yc de(s) l'autre(s) composé(s) organo-iodé(s):
- à la fin de l'étape a), le composé intermédiaire Ya n'est pas séparé de l'AATI éventuellement présent dans le milieu réactionnel à la fin de l'étape a), et/ou
- à la fin de l'étape b), le composé intermédiaire Yb n'est pas séparé du composé intermédiaire Ya éventuellement présent dans le milieu réactionnel à la fin de l'étape b), et/ou
- à la fin de l'étape c), le composé intermédiaire Yc n'est pas séparé du composé intermédiaire Yb éventuellement présent dans le milieu réactionnel à la fin de l'étape c.

### Procédé de préparation d'un composé organo-iodé

Selon un mode de réalisation, les étapes a) d'acylation, b) de chloruration, c) d'amidification et d) de déprotection du procédé selon l'invention sont réalisées sans isolement des composés Ya et Yc dans un seul réacteur ou dans plusieurs réacteurs, préférentiellement dans un seul réacteur.

Selon un mode de réalisation, l'étape a) d'acylation de l'acide 2,4,6-triiodo-5-aminoisophtalique de formule (A) suivante : 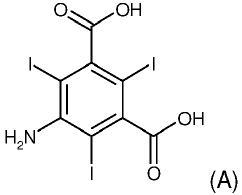 est réalisée par un chlorure d'acyle, préférentiellement par le chlorure de chloroacétyle (CAC) ou le chlorure d'acétoxyacétyle (AAC), plus préférentiellement par le chlorure d'acétoxyacétyle.
L'AAC a comme avantage de ne pas être considéré comme un composé toxique et cancérogène, mutagène ou toxique pour la reproduction (CMR) et il agit très rapidement en tant qu'agent acylant.

Selon un mode de réalisation particulier, le procédé selon l'invention comprend les étapes suivantes :
a) acylation de l'acide 2,4,6-triiodo-5-aminoisophtalique de formule (A) suivante : 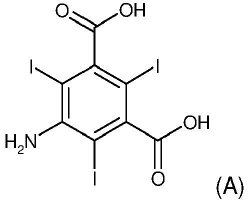
   par un composé de formule générale (I) suivante :

      R₂-C(O)Cl (I),
   R₂ étant un groupe -CH₂-GP dans lequel GP est un groupe partant choisi parmi un halogénure, un acétate, un mésylate, un tosylate et un triflate, R₂ étant de préférence choisi parmi -CH₂Cl et 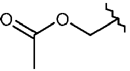
   pour obtenir un composé intermédiaire Ya ;
   puis
b) chloruration du composé intermédiaire Ya pour obtenir un composé intermédiaire organo-iodé Yb de formule générale (II) suivante : 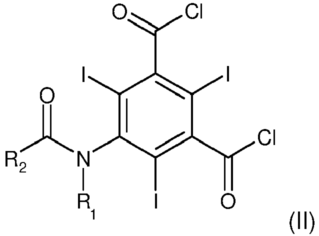
   R₁ étant H ou un groupe méthyle, de préférence H, et
   R₂ étant tel que défini ci-dessus, puis
c) amidification du composé intermédiaire organo-iodé Yb, en présence d'une base inorganique, pour obtenir un composé intermédiaire Yc ;
   puis
d) déprotection du composé intermédiaire Yc,
   les étapes a), b), c) et d) étant effectuées sans isolement des composés intermédiaires Ya et Yc.

L'étape c) est généralement réalisée en présence d'une amine de formule (IV) :

R₃-NH₂ (IV),

dans laquelle R₃ représente un groupe alkyle comprenant de 1 à 6 atomes de carbone, notamment de 2, 3 ou 4 atomes de carbone, ledit groupe alkyle étant éventuellement substitué par un ou plusieurs groupes hydroxyle, de préférence deux groupes hydroxyle, ou un sel de celle-ci. L'amine préférée est l'aminopropanediol.

De préférence, la quantité d'amine de formule (IV) utilisée lors de l'étape c) est telle que le ratio molaire de l'amine de formule (IV) par rapport à l'AATI utilisé lors de l'étape a) est de 1,5 à 4,0, notamment de 1,8 à 3,0, et préférentiellement de 2,0 à 2,3.

Le composé organo-iodé Yd obtenu à la fin de l'étape d), de préférence le composé de formule générale (V) telle que définie ci-dessous, est utile en tant qu'intermédiaire de synthèse du produit de contraste ioversol. 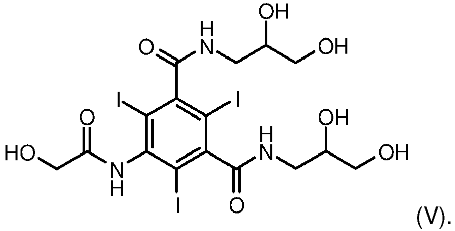 Le ioversol est vendu sous le nom de marque Optiject^{®} ou Optiray^{®} et a la formule chimique suivante :

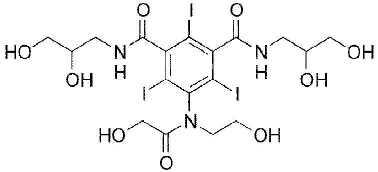

Selon un mode de réalisation, les étapes a), b), c) et/ou d) sont réalisées en présence d'un solvant aprotique et polaire.

Selon un mode de réalisation, les étapes a), b), c) et/ou d) sont réalisées en présence d'au moins un solvant choisi parmi le groupe constitué du diméthylacétamide, du carbonate de propylène, de l'acétonitrile et du tétrahydrofurane ou d'un mélange de ceux-ci. De préférence, le solvant comprend un mélange de diméthylacétamide et de carbonate de propylène. En particulier, pour les raisons détaillées ci-dessus, il est avantageux que le solvant de l'étape b) soit un mélange de carbonate de propylène et de diméthylacétamide.

Dans un mode de réalisation préféré :
- le solvant de l'étape a) est du diméthylacétamide, et/ou
- le solvant de l'étape b) est un mélange de carbonate de propylène et de diméthylacétamide, et/ou
- le solvant de l'étape c) et/ou de l'étape d) est un mélange de diméthylacétamide et d'une solution aqueuse.

Les étapes a), b), c) et d) étant de préférence réalisées de façon monotope, l'étape b) est réalisée dans le milieu réactionnel résultant de l'étape a), l'étape c) est réalisée dans le milieu réactionnel résultant de l'étape b) et l'étape d) réalisée dans le milieu réactionnel résultant de l'étape c) : le(s) solvant(s) utilisé(s) ainsi que leur(s) quantité(s) sont donc préférentiellement identique(s). Selon un mode de réalisation, lors de l'étape b), un ou plusieurs solvant(s) peu(ven)t être ajouté(s) à celui(ceux) utilisé(s) pour l'étape a) et/ou lors de l'étape c), un ou plusieurs solvant(s) peu(ven)t être ajouté(s) à celui(ceux) utilisé(s) pour l'étape b) et/ou lors de l'étape d), un ou plusieurs solvant(s) peu(ven)t être ajouté(s) à celui(ceux) utilisé(s) pour l'étape c).

Selon un autre mode de réalisation, le ratio, en litre par kilogramme, entre la quantité de solvant (en litre) et la quantité d'acide 2,4,6-triiodo-5-aminoisophtalique (en kg) est compris entre 5 pour 1 et 1 pour 1, de préférence de 3 pour 1 ou 2,5 pour 1. De tels ratios permettent avantageusement de dissoudre la totalité du composé intermédiaire Yb, ce qui permet d'optimiser les étapes c) et d) ultérieures.

Généralement, le composé organo-iodé obtenu à la fin de l'étape d) a la formule (VI) suivante : 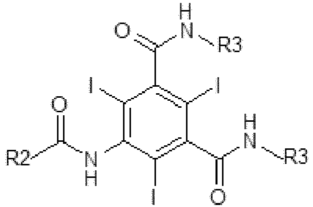 dans laquelle R₂ et R₃ sont tels que définis ci-dessus.

Les étapes a), b), c) et d) peuvent éventuellement être suivies d'une étape e) d'alkylation du composé obtenu à la fin de l'étape d) avec un agent alkylant, notamment l'oxyde d'éthylène ou un agent alkylant de formule (VII) :

R₄-GP' (VII),

dans laquelle :
- R₄ réprésente un groupe alkyle comprenant de 1 à 6 atomes de carbone, notamment de 2, 3 ou 4 atomes de carbone, ledit groupe alkyle étant éventuellement substitué par un ou plusieurs groupes hydroxyle, et
- GP' est un groupe partant, notamment choisi parmi un halogénure, un mésylate, un tosylate et un triflate,
L'agent alkylant est typiquement du 2-chloroéthanol ou de l'oxyde d'éthylène, et l'étape e) conduit à la formation de l'ioversol.

Les modes de réalisation décrits ci-dessous pour chacune des étapes a) à d) peuvent être combinés entre eux.

### Etape a) d'acylation

Selon un mode de réalisation, l'acylation de l'étape a) est effectuée en présence d'un agent acylant choisi parmi le chlorure de chloroacétyle et le chlorure d'acétoxyacétyle.

Ces agents acylants ont les formules chimiques suivantes :

| **Nom de l'agent acylant** | **Numéro CAS** | **Formule chimique** |
|---|---|---|
| Chlorure de chloroacétyle | 79-04-9 | 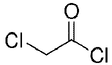 |
| Chlorure d'acétoxyacétyle | 13831-31-7 | 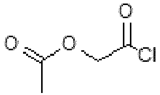 |

Selon un mode de réalisation, l'acylation de l'étape a) est effectuée en présence d'un agent acylant, de préférence un chlorure d'acyle, présent en une quantité comprise entre 1 et 1,5 équivalents molaires par rapport à la quantité d'acide 2,4,6-triiodo-5-aminoisophtalique ; préférentiellement entre 1,1 et 1,3, par exemple 1,1 ou 1,3 équivalent molaire par rapport à la quantité d'acide 2,4,6-triiodo-5-aminoisophtalique.

### Etape b) de chloruration

Selon un mode de réalisation, la chloruration de l'étape b) est effectuée en présence d'un agent chlorurant choisi parmi le groupe constitué du chlorure de thionyle, de l'oxychlorure de phosphore, du trichlorure de phosphore, du chlorure d'oxalyle, du pentachlorure de phosphore, du dichlorure de méthanoyle et d'un mélange de ceux-ci. Selon un mode de réalisation, la chloruration de l'étape b) est effectuée en présence d'un réactif choisi parmi le groupe constitué du chlorure de thionyle, du trichlorure de phosphore, du pentachlorure de phosphore et d'un mélange de ceux-ci. De préférence, l'agent chlorurant est le chlorure de thionyle, car les dérivés phosphorés mentionnés ci-dessus génèrent des sels de phosphate qui sont plus polluants et dont l'élimination complique le procédé.

Selon un mode de réalisation particulier, la quantité d'agent chlorurant est comprise entre 2 et 6 équivalents molaires par rapport à la quantité d'acide 2,4,6-triiodo-5-aminoisophtalique, préférentiellement comprise entre 2,5 et 5 équivalents, plus préférentiellement entre 3 et 5 équivalents, par exemple 3,5 ou 5 équivalents, encore plus préférentiellement entre 3,2 et 4 équivalents, par rapport à la quantité d'acide 2,4,6-triiodo-5-aminoisophtalique.

Selon un mode de réalisation, l'étape a) est réalisée pendant une durée de 2 à 70 heures, préférentiellement de 2 à 24h ; et/ou l'étape b) est réalisée pendant une durée de 2 à 22 heures, préférentiellement de 4 à 12h.

L'étape a) est typiquement réalisée à une température de 10 à 70°C, de préférence de 15 à 60°C, encore plus préférentiellement de 30 à 60°C, notamment de 45 à 55°C, par exemple à 50°C. Quand la température est trop basse, cela diminue le rendement de l'étape a), et donc du procédé, et quand elle est trop haute, il y a génération de plus d'impuretés, donc diminution de la pureté. Les gammes de températures ci-dessus sont optimales pour réduire l'apparition de certaines impuretés tout en maintenant une durée de réaction économiquement acceptable.

L'étape b) est de préférence réalisée à une température de -15 à 30°C, préférentiellement de -10 à 10°C, encore plus préférentiellement de 0 à 10°C.

### Etape c) d'amidification

Selon un mode de réalisation, l'amidification de l'étape c) est effectuée en présence d'aminopropanediol (aussi appelée APD).

L'amidification de l'étape c) est effectuée en présence d'une base inorganique, par exemple de la soude (NaOH). Les bases inorganiques sont généralement moins toxiques que certaines bases organiques telles que la triéthylamine. En outre, utiliser une base organique comprenant une fonction amine requiert d'ajouter une étape d'élimination du sel d'ammonium formé à partir de cette base organique, ce qui complexifie le procédé et rend délicate la mise en oeuvre du procédé sans isoler le composé intermédiaire Yc. L'utilisation de base inorganique telle que NaOH génère des sels, tels que NaCl, qui sont moins solubles dans le milieu réactionnel obtenu à la fin de l'étape b) et donc qui seront plus facilement éliminables du milieu réactionnel.

L'étape c) est généralement mise en oeuvre en présence d'une solution aqueuse, typiquement d'une solution aqueuse d'une base inorganique, comme une solution aqueuse de NaOH. Il y avait un préjugé technique à mettre en oeuvre l'étape c) en présence d'une solution aqueuse, car l'homme du métier se serait attendu à la dégradation des fonctions chlorure d'acyle du composé intermédiaire Yb en présence d'une telle solution, dégradation qui conduirait à reformer le composé intermédiaire Ya. L'eau est connue pour être néfaste pour l'amidification en partant d'un chlorure d'acyle.

L'étape c) est généralement réalisée à une température de 5 à 30°C, préférentiellement de 5 à 20°C, encore plus préférentiellement de 5 à 15°C. De telles températures permettent notamment de réduire la teneur en impuretés polaires (par exemple, des impuretés mono-amidifiées).

### Etape d) de déprotection

L'homme du métier est à même de déterminer au vu de ses connaissances générales illustrée par l'ouvrage « Greene's Protective Groups in Organic Synthesis » de Wiley (ISBN-13: 978-0471697541) comment déprotéger en fonction de la nature du groupe protecteur et donc de l'agent acylant utilisé à l'étape a).

Selon un mode de réalisation, la déprotection de l'étape d) est effectuée d'abord par l'ajout d'une base puis par l'ajout d'un acide. Préférentiellement, on utilisera une base choisie parmi les bases organiques ou les bases inorganiques. Plus préférentiellement, on utilisera une base organique choisie parmi un mélange de triéthylamine et de méthanol, un mélange de pyridine et d'eau et leurs mélanges. Plus préférentiellement, on utilisera une base inorganique choisie parmi la soude, de l'ammoniaque aqueux, un mélange d'ammoniaque et de toluène, du carbonate de potassium (K₂CO₃), un mélange d'hydrazine et de méthanol et leurs mélanges. Préférentiellement, on utilisera un acide inorganique. Plus préférentiellement, on utilisera un acide inorganique choisi parmi l'acide chlorhydrique, l'acide phosphorique, l'acide sulfurique et leurs mélanges.

Selon un mode de réalisation, la déprotection de l'étape d) est effectuée d'abord par l'ajout d'une base puis par l'ajout d'un acide suivi de l'ajout d'un solvant. Le solvant est préférentiellement choisi parmi l'isopropanol, l'éthanol, le méthanol et leurs mélanges, plus préférentiellement l'isopropanol, l'éthanol et leurs mélanges.

Selon un mode de réalisation, la déprotection de l'étape d) est effectuée d'abord par l'ajout d'une base comme une base inorganique, typiquement d'un mélange de soude et d'eau puis par l'ajout d'un acide comme un acide inorganique tel que l"acide chlorhydrique, éventuellement suivi par l'ajout d'isopropanol. Ce mode de réalisation est particulièrement adapté lorsque l'agent acylant de l'étape a) était le chlorure d'acétoxyacétyle. On déprotège ainsi le groupement acétyle sur le composé obtenu à l'étape c). Préférentiellement, la déprotection de l'étape d) est effectuée à une température de 15 à 70°C, préférentiellement de 20 à 60°C, encore plus préférentiellement de 30 à 55°C.

Lorsque le chlorure de chloroacétyle est utilisé comme agent acylant à l'étape a), la déprotection de l'étape d) est effectuée par
- ajout d'une solution aqueuse acide, telle qu'une solution aqueuse d'acide chlorhydrique, typiquement jusqu'à un pH de 3,0 à 4,5, mise sous pression à une pression de 1,05 à 1,5 bars, par exemple 1,2 bars et chauffage à une température comprise entre 110 et 130°C, et du pH du milieu réactionnel entre 3,0 et 4,5 par ajout d'une base, par exemple de la soude, puis
- ajout d'une base, par exemple une solution aqueuse de NaOH, jusqu'à ce que le pH du milieu réactionnel atteigne 4,5 à 5,5.

Les exemples figurant ci-après sont présentés à titre illustratif et ne sont pas limitatifs de l'invention.

### EXEMPLES

Par « V », on entend désigner un rapport volumique, soit le volume d'un réactif ou d'un solvant par rapport à un 1 kg d'AATI.

Par « eq. », on entend désigner un nombre d'équivalent molaire, soit le rapport entre le nombre de moles d'un réactif et le nombre de moles de l'AATI.

### Exemple 1 : Synthèse selon l'invention d'un intermédiaire de synthèse du ioversol

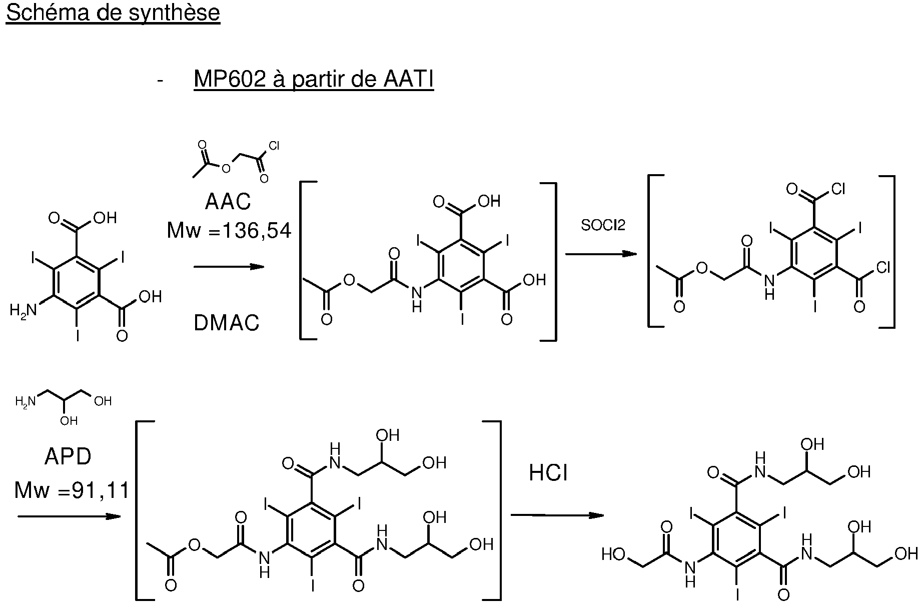

### Etape d'acylation :

Du 2-chloro-2-oxoéthyl acétate aussi appelé AAC (281g, 1.15 eq.) est dissous dans du diméthylacetamide (DMAC) (1.35L) et l'AATI (1kg, 1.0 eq.) est graduellement ajouté à 50°C. Le mélange réactionnel est mélangé pendant 6 heures à 50°C. Du carbonate de propylène (1.15L) est ajouté et la réaction est refroidie à 5°C.

### Etape de chloruration :

Du chlorure de thionyle SOCl₂ (745g, 3.5 eq.) est versé à 5°C pendant 2 heures et le milieu réactionnel est mélangé 5h à 5°C.

Le milieu réactionnel est versé dans une solution aqueuse d'acétate de sodium (AcONa) de façon à précipiter l'intermédiaire de synthèse « DiCOA like ». La suspension est filtrée et la partie solide est re-dissoute dans du DMAC (1,5 mL) pour obtenir une solution.

### Etape d'amidification :

Un mélange d'aminopropanediol (APD) (359g, 2.2 eq.) et d'une solution aqueuse de soude (NaOH) à 30% (359 ml) est versé dans la solution pendant 6 heures. Le milieu réactionnel est mélangé 2 heures à 12.5°C.

### Etape de déprotection :

De l'eau (1L) et une solution aqueuse de soude (NaOH) 30% (359 ml) sont ajoutées à 50°C durant 2h30. De l'acide chlorhydrique (HCl) à 37% (900 ml) est ensuite ajouté à 50°C et la réaction est refroidie à 5°C. La suspension est filtrée, lavée avec un mélange isopropanol (IPA)/eau et séchée. Le rendement obtenu est de 83% et la pureté obtenue est de 99%.

### Exemple 2 : synthèse du ioversol à partir de l'intermédiaire de synthèse obtenu selon l'invention

Le composé obtenu à l'exemple 1 est ensuite alkylé en utilisant du 2-chloroéthanol ou de l'oxyde d'éthylène. Le composé ainsi obtenu est ensuite purifié et séché afin d'obtenir le ioversol.

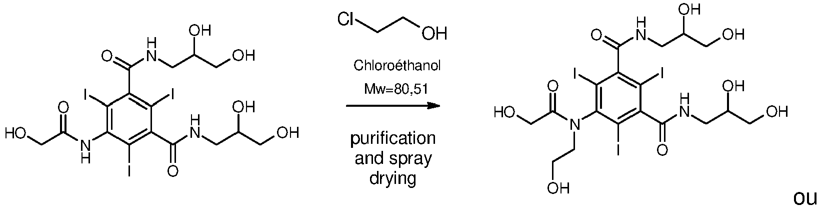

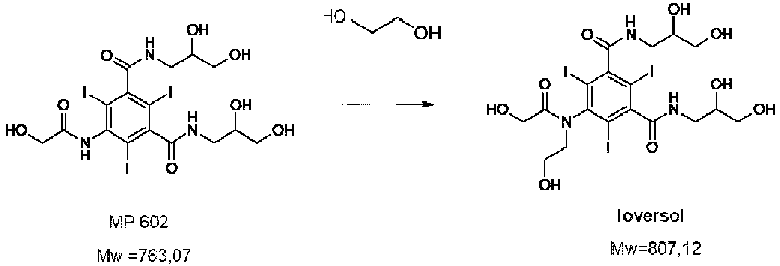

## Revendications

1. Procédé de préparation d'un composé organo-iodé comprenant les étapes suivantes :
a) acylation de l'acide 2,4,6-triiodo-5-aminoisophtalique de formule (A) suivante : 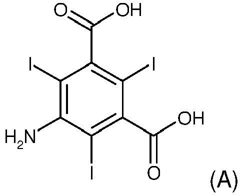
pour obtenir un composé intermédiaire Ya ;
puis
b) chloruration du composé intermédiaire Ya pour obtenir un composé intermédiaire organo-iodé Yb ;
puis
c) amidification du composé intermédiaire organo-iodé Yb, en présence d'une base inorganique, pour obtenir un composé intermédiaire Yc ;
puis
d) déprotection du composé intermédiaire Yc,
les étapes a), b), c) et d) étant effectuées sans isolement des composés Ya et Yc.

2. Procédé de préparation selon la revendication 1, comprenant les étapes suivantes :
a) acylation de l'acide 2,4,6-triiodo-5-aminoisophtalique de formule (A) suivante : 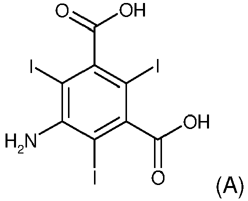
par un composé de formule générale (I) suivante :
R₂-C(O)CI (I),
R₂ étant un groupe -CH₂-GP dans lequel GP est un groupe partant choisi parmi un halogénure, un acétate, un mésylate, un tosylate et un triflate,
pour obtenir un composé intermédiaire Ya ;
puis
b) chloruration du composé intermédiaire Ya pour obtenir un composé intermédiaire organo-iodé Yb de formule générale (II) suivante : 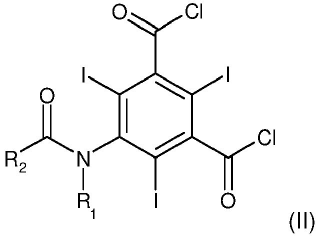
R₁ étant H ou un groupe méthyle, de préférence H, et
R₂ étant tel que défini ci-dessus,
puis
c) amidification du composé intermédiaire organo-iodé Yb, en présence d'une base inorganique, pour obtenir un composé intermédiaire Yc ;
puis
d) déprotection du composé intermédiaire Yc,
les étapes a), b), c) et d) étant effectuées sans isolement des composés Ya et Yc.

3. Procédé de préparation selon la revendication 1 ou 2, dans lequel R₂ est choisi parmi -CH₂Cl et

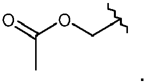

4. Procédé de préparation selon l'une quelconque des revendications 1 à 3, dans lequel les étapes a), b), c) et d) sont effectuées sans purification du composé intermédiaire Yb.

5. Procédé de préparation selon l'une quelconque des revendications 1 à 4, exempt d'ajout d'un non-solvant du composé intermédiaire Ya pendant l'étape a) et entre les étapes a) et b).

6. Procédé de préparation selon l'une quelconque des revendications 1 à 5, dans lequel les étapes a), b) c) et d) sont réalisées dans un seul réacteur.

7. Procédé de préparation selon l'une quelconque des revendications précédentes, dans lequel les étapes a), b), c) et d) sont réalisées en présence d'au moins un solvant choisi parmi le groupe constitué du diméthylacétamide, du carbonate de propylène, de l'acétonitrile et du tétrahydrofurane ou d'un mélange de ceux-ci.

8. Procédé de préparation selon l'une quelconque des revendications précédentes, dans lequel le solvant de l'étape b) est un mélange de carbonate de propylène et de diméthylacétamide.

9. Procédé de préparation selon l'une quelconque des revendications précédentes, dans lequel l'étape c) est mise en oeuvre en présence d'une solution aqueuse, notamment d'une solution aqueuse d'une base inorganique, de préférence une solution aqueuse de NaOH.

10. Procédé de préparation selon l'une quelconque des revendications 7 à 9, dans lequel :
- le solvant de l'étape a) est du diméthylacétamide, et/ou
- le solvant de l'étape c) et/ou de l'étape d) est un mélange de diméthylacétamide et d'une solution aqueuse.

11. Procédé de préparation selon l'une quelconque des revendications précédentes, dans lequel la chloruration de l'étape b) est effectuée en présence d'un agent chlorurant choisi parmi le groupe constitué du chlorure de thionyle, de l'oxychlorure de phosphore, du trichlorure de phosphore, du chlorure d'oxalyle, du pentachlorure de phosphore, du dichlorure de méthanoyle et d'un mélange de ceux-ci.

12. Procédé de préparation selon l'une quelconque des revendications précédentes, comprenant, à la fin de l'étape b) :
- l'ajout d'un non-solvant du composé intermédiaire Yb dans le milieu réactionnel obtenu à la fin de l'étape b), ce par quoi le composé intermédiaire Yb précipite dans le milieu réactionnel et un milieu réactionnel comprenant une phase liquide et un précipité est obtenu,
- la séparation du précipité et de la phase liquide, puis
- la dissolution du précipité dans un solvant du composé intermédiaire Yb pour obtenir une solution qui est utilisée pour mettre en oeuvre l'étape c).

13. Procédé de préparation selon l'une quelconque des revendications précédentes, dans lequel l'amidification de l'étape c) est effectuée avec de l'aminopropanediol.

14. Procédé de préparation selon l'une quelconque des revendications précédentes, dans lequel la déprotection de l'étape d) est effectuée avec de la soude, de l'acide chlorhydrique ou un mélange de ceux-ci.

## Patentansprüche

1. Verfahren zur Herstellung einer Organoiodverbindung, das die folgenden Schritte umfasst:
a) Acylierung von 2,4,6-Triiod-5-aminoisophthalsäure der folgenden Formel (A): 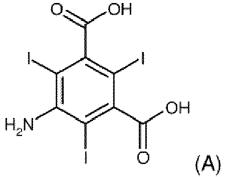
unter Erhalt einer Zwischenverbindung Ya;
dann
b) Chlorierung der Zwischenverbindung Ya unter Erhalt einer Organoiodzwischenverbindung Yb; dann
c) Amidierung der Organoiodzwischenverbindung Yb in Gegenwart einer anorganischen Base unter Erhalt einer Zwischenverbindung Yc;
dann
d) Entschützung der Zwischenverbindung Yc,
wobei die Schritte a), b), c) und d) ohne Isolierung der Verbindungen Ya und Yc durchgeführt werden.

2. Herstellungsverfahren nach Anspruch 1, das die folgenden Schritte umfasst:
a) Acylierung von 2,4,6-Triiod-5-aminoisophthalsäure der folgenden Formel (A): 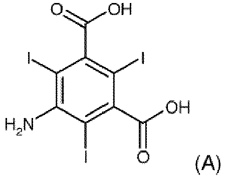
mit einer Verbindung der folgenden allgemeinen Formel (I):
R₂-C(O)Cl (I),
wobei R₂ für eine Gruppe -CH₂-GP steht, wobei GP für eine Abgangsgruppe steht, die aus einem Halogenid, einem Acetat, einem Mesylat, einem Tosylat und einem Triflat ausgewählt ist,
unter Erhalt einer Zwischenverbindung Ya;
dann
b) Chlorierung der Zwischenverbindung Ya unter Erhalt einer Organoiodzwischenverbindung Yb der folgenden allgemeinen Formel (II): 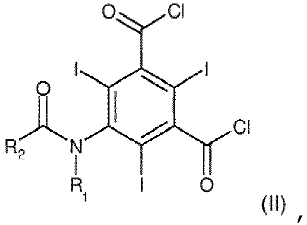
wobei R₁ für H oder eine Methylgruppe, bevorzugt H, steht und
R₂ wie oben definiert ist,
dann
c) Amidierung der Organoiodzwischenverbindung Yb in Gegenwart einer anorganischen Base unter Erhalt einer Zwischenverbindung Yc;
dann
d) Entschützung der Zwischenverbindung Yc,
wobei die Schritte a), b), c) und d) ohne Isolierung der Verbindungen Ya und Yc durchgeführt werden.

3. Herstellungsverfahren nach Anspruch 1 oder 2, wobei R₂ aus -CH₂Cl und 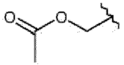 ausgewählt ist.

4. Herstellungsverfahren nach einem der Ansprüche 1 bis 3, wobei die Schritte a), b), c) und d) ohne Reinigung der Zwischenverbindung Yb durchgeführt werden.

5. Herstellungsverfahren nach einem der Ansprüche 1 bis 4 ohne Zugabe eines Nichtlösungsmittels der Zwischenverbindung Ya während Schritt a) und zwischen den Schritten a) und b).

6. Herstellungsverfahren nach einem der Ansprüche 1 bis 5, wobei die Schritte a), b), c) und d) in einem einzigen Reaktor durchgeführt werden.

7. Herstellungsverfahren nach einem der vorhergehenden Ansprüche, wobei die Schritte a), b), c) und d) in Gegenwart von mindestens einem Lösungsmittel aus der Gruppe bestehend aus Dimethylacetamid, Propylencarbonat, Acetonitril und Tetrahydrofuran oder einer Mischung davon durchgeführt werden.

8. Herstellungsverfahren nach einem der vorhergehenden Ansprüche, wobei es sich bei dem Lösungsmittel von Schritt b) um eine Mischung von Propylencarbonat und Dimethylacetamid handelt.

9. Herstellungsverfahren nach einem der vorhergehenden Ansprüche, wobei der Schritt c) in Gegenwart einer wässrigen Lösung, insbesondere einer wässrigen Lösung einer anorganischen Base, vorzugsweise einer wässrigen Lösung von NaOH, durchgeführt wird.

10. Herstellungsverfahren nach einem der Ansprüche 7 bis 9, wobei:
- es sich bei dem Lösungsmittel von Schritt a) um Dimethylacetamid handelt und/oder
- es sich bei dem Lösungsmittel von Schritt c) und/oder Schritt d) um eine Mischung von Dimethylacetamid und einer wässrigen Lösung handelt.

11. Herstellungsverfahren nach einem der vorhergehenden Ansprüche, wobei die Chlorierung von Schritt b) in Gegenwart eines Chlorierungsmittels aus der Gruppe bestehend aus Thionylchlorid, Phosphoroxidchlorid, Phosphortrichlorid, Oxalylchlorid, Phosphorpentachlorid, Methanoyldichlorid und einer Mischung davon durchgeführt wird.

12. Herstellungsverfahren nach einem der vorhergehenden Ansprüche, umfassend am Ende von Schritt b):
- die Zugabe eines Nichtlösungsmittels der Zwischenverbindung Yb zu dem am Ende von Schritt b) erhaltenen Reaktionsmedium, wodurch die Zwischenverbindung Yb aus dem Reaktionsmedium ausfällt und ein Reaktionsmedium, das eine flüssige Phase und einen Niederschlag umfasst, erhalten wird,
- die Trennung des Niederschlags und der flüssigen Phase, dann
- die Auflösung des Niederschlags in einem Lösungsmittel der Zwischenverbindung Yb unter Erhalt einer Lösung, die für die Durchführung von Schritt c) verwendet wird.

13. Herstellungsverfahren nach einem der vorhergehenden Ansprüche, wobei die Amidierung von Schritt c) mit Aminopropandiol durchgeführt wird.

14. Herstellungsverfahren nach einem der vorhergehenden Ansprüche, wobei die Entschützung von Schritt b) mit Natriumcarbonat, Salzsäure oder einer Mischung davon durchgeführt wird.

## Claims

1. A process for preparing an organo-iodinated compound, comprising the following steps:
a) acylation of 2,4,6-triiodo-5-aminoisophthalic acid of formula (A) below: 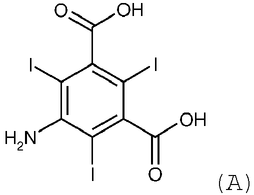 in order to obtain an intermediate compound Ya; then
b) chlorination of the intermediate compound Ya in order to obtain an organo-iodinated intermediate compound Yb;
then
c) amidation of the organo-iodinated intermediate compound Yb, in the presence of an inorganic base, in order to obtain an intermediate compound Yc; then
d) deprotection of the intermediate compound Yc,
the steps a), b), c) and d) being carried out without isolation of the compounds Ya and Yc.

2. Preparation process according to Claim 1, comprising the following steps:
a) acylation of 2,4,6-triiodo-5-aminoisophthalic acid of formula (A) below: 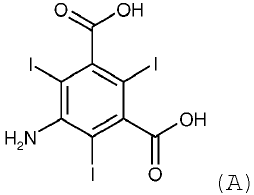
by a compound of general formula (I) below:
R₂-C(O)Cl (I),
R₂ being a -CH₂-GP group wherein GP is a leaving group chosen from a halide, an acetate, a mesylate, a tosylate and a triflate,
in order to obtain an intermediate compound Ya;
then
b) chlorination of the intermediate compound Ya in order to obtain an organo-iodinated intermediate compound Yb of general formula (II) below: 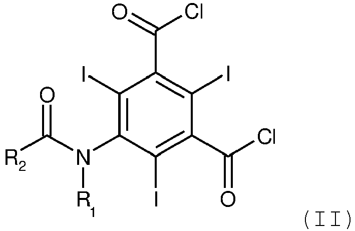
R₁ being H or a methyl group, preferably H, and
R₂ being as defined above,
then
c) amidation of the organo-iodinated intermediate compound Yb, in the presence of an inorganic base, in order to obtain an intermediate compound Yc; then
d) deprotection of the intermediate compound Yc,
the steps a), b), c) and d) being carried out without isolation of the compounds Ya and Yc.

3. Preparation process according to Claim 1 or 2, wherein R₂ is chosen from -CH₂Cl and

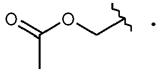

4. Preparation process according to any one of Claims 1 to 3, wherein the steps a), b), c) and d) are carried out without purification of the intermediate compound Yb.

5. Preparation process according to any one of Claims 1 to 4, free of addition of a nonsolvent of the intermediate compound Ya during step a) and between steps a) and b).

6. Preparation process according to any one of Claims 1 to 5, wherein the steps a), b), c) and d) are carried out in a single reactor.

7. Preparation process according to any one of the preceding claims, wherein the steps a), b), c) and d) are carried out in the presence of at least one solvent chosen from the group consisting of dimethylacetamide, propylene carbonate, acetonitrile and tetrahydrofuran or a mixture thereof.

8. Preparation process according to any one of the preceding claims, wherein the solvent of step b) is a mixture of propylene carbonate and dimethylacetamide.

9. Preparation process according to any one of the preceding claims, wherein step c) is carried out in the presence of an aqueous solution, notably an aqueous solution of an inorganic base, preferably an aqueous solution of NaOH.

10. Preparation process according to any one of Claims 7 to 9, wherein:
- the solvent of step a) is dimethylacetamide, and/or
- the solvent of step c) and/or of step d) is a mixture of dimethylacetamide and an aqueous solution.

11. Preparation process according to any one of the preceding claims, wherein the chlorination of step b) is carried out in the presence of a chlorinating agent chosen from the group consisting of thionyl chloride, phosphorus oxychloride, phosphorus trichloride, oxalyl chloride, phosphorus pentachloride, methanoyl dichloride and a mixture thereof.

12. Preparation process according to any one of the preceding claims, comprising, at the end of step b):
- the addition of a nonsolvent of the intermediate compound Yb into the reaction medium obtained at the end of step b), whereby the intermediate compound Yb precipitates from the reaction medium and a reaction medium comprising a liquid phase and a precipitate is obtained,
- the separation of the precipitate and the liquid phase, then
- the dissolving of the precipitate in a solvent of the intermediate compound Yb in order to obtain a solution which is used to carry out step c).

13. Preparation process according to any one of the preceding claims, wherein the amidation of step c) is carried out with aminopropanediol.

14. Preparation process according to any one of the preceding claims, wherein the deprotection of step d) is carried out with sodium hydroxide, hydrochloric acid or a mixture thereof.
